# EUROPEAN PATENT APPLICATION

(11) **EP 4 491 117 A1**
(43) Date of publication of application: **15.01.2025**
(21) Application number: 23184436.6
(22) Date of filing: 10.07.2023
(51) Int. Cl.: A61B 5/1491

(54) **TISSUE IMAGING WITH THERMAL PROVOCATION**

(71) Applicant: Neko Health AB, 115 45 Stockholm (SE)
(72) Inventor: WINDÅ, Mattias, 185 39 Vaxholm (SE); ARVIDSSON, Magnus, 192 70 Sollentuna (SE); SALOMONSSON, Fredrik, 182 64 Djursholm (SE)
(74) Representative: Kransell & Wennborg KB

(57) **Abstract**

The present disclosure relates to studying hemodynamics in live skin tissue of a subject. A first imaging of a target area (1) of the skin is performed using a tissue imaging system (10) to generate first imaging data. In parallel, at each of at least two sites (13) within said target area, thermal provocation is performed, including heating the skin of the site to a predetermined temperature. During or after the thermal provocation, a second imaging of the target area is performed using said tissue imaging system to generate second imaging data. Then, a skin blood circulation response to the thermal provocation in the target area is determined based on the first and second imaging data.

## Description

### TECHNICAL FIELD

The present disclosure relates to studying hemodynamics in live skin tissue of a subject.

### BACKGROUND

Vascular diseases, such as arteriosclerosis, media-sclerosis, and other pathologies, present a significant challenge for diagnosis and treatment. The diagnostic methods currently available for these conditions are typically invasive, presenting risks to the patient and limiting their suitability for widespread screening. Furthermore, these methods generally provide limited information, which hampers the ability of medical practitioners to make informed decisions regarding treatment and management of the underlying pathology.

Several prior art methods and devices have been developed to attempt to address these issues. For example, the Perimed Periflux 6000 EPOS^{™} system offers heat provocation at a single spot, but the results are highly dependent on the specific location chosen, and the method does not provide comprehensive imaging data. The Modulim Clarify^{™} system offers imaging of hemoglobin but does not include provocation or thermal imaging, limiting its applications.

These prior art systems all have significant shortcomings, including the inability to provide robust, comprehensive, and non-invasive diagnostic data suitable for widespread screening of vascular pathologies. Given the spatial variations in skin composition and the sensitivity of the skin micro-circulation to factors like ambient temperature, there is a clear need for a diagnostic system that combines quantitative imaging of vascular properties with heat provocation, enabling more accurate and effective assessment of vascular diseases and conditions.

### SUMMARY

It is an objective of the present invention to provide improved ways of studying hemodynamics in live skin tissue of a subject. This is achieved by studying the live tissue before and after heat provocation.

According to an aspect of the present invention, there is provided a method for studying hemodynamics in live skin tissue of a subject. The method comprises performing a first imaging of a target area of the skin using a tissue imaging system to generate first imaging data. The method also comprises, in parallel, at each of at least two sites within said imaged target area, performing thermal provocation including heating the skin of the site to a predetermined temperature. The method also comprises, during or after the thermal provocation, performing a second imaging of the target area using said tissue imaging system to generate second imaging data. The method also comprises determining a skin blood circulation response to the thermal provocation in the target area based on the first and second imaging data.

According to another aspect of the present invention, there is provided a tissue imaging system for performing an embodiment of the method of the present disclosure. The system comprises a tissue imaging device for performing the imaging of the target area, a thermal provocation device for, in parallel, at each of the at least two sites within the target area, performing the thermal provocation, and a controller comprising processing circuitry and storage storing instructions executable by said processing circuitry whereby said controller is operative to determine the skin blood circulation response to the thermal provocation in the target area.

By heating the skin to a predetermined temperature, responses of the skin to the heat provocation can be studied in a controlled manner. It is generally regarded as safe to heat the skin tissue to a predetermined temperature of 43°C. The predetermined temperature may thus be within the range of 35-43°C. The skin blood circulation may consist of microcirculation including capillaries, arterioles and venules in the skin.

In some embodiments, the respective predetermined temperature of each of the at least two sites are different to each other. For instance, the predetermined temperature of a first one of the sites may be 35°C and the predetermined temperature of a second of the sites may be 40°C. By using two or more predetermined temperature, one per site, a more complete study of the response to heat provocation may be achieved, and also time may be saved compared to running two temperatures sequentially in one site.

In some embodiments, the thermal provocation also includes regulating the skin temperature of the site to remain at said predetermined temperature for the duration of the thermal provocation. Thus, the temperature at each site is not merely heated to the predetermined temperature but also kept at said predetermined temperature for a predetermined time period, e.g. to enable performing the second imaging during the thermal provocation. To enable performing the second imaging during the thermal provocation, the sites must be heated in a way which does not prevent concurrent imaging of the sites, e.g. by focused infrared radiation to the sites or by a transparent heating device, as discussed below. Pre-heated metal lumps may then not be suitable, but may be used if the second imaging is performed after the thermal provocation, i.e. after removal of the metal lumps. In some embodiments,

In some embodiments, the regulating is based on real-time thermal data from a thermal camera detecting the skin temperature of each site. The thermal camera may be part of the tissue imaging system and may be a convenient way of detecting the skin temperature in real time, e.g. during heat provocation by infrared light, or directly after heat provocation.

Additionally or alternatively, in some embodiments, thermal data from a thermal camera, possibly the same thermal camera which may be used for detecting the skin temperature of each site, is used for selecting the target area and/or the at least two sites, prior to performing the first imaging. By means of the thermal camera, suitable sites may be found, e.g. away from larger blood vessels.

Additionally or alternatively, in some embodiments, thermal data from a thermal camera, possibly the same thermal camera which may be used for detecting the skin temperature of each site and/or for selecting the target area and/or the at least two sites, is used for normalizing a blood property derivable from the first and second imaging data, with regard to temperature, e.g. wherein the property is at least one of perfusion, hemoglobin concentration, oxygenation. Thus, the skin blood circulation response with regard to said property/-ies may be made independent of e.g. the ambient temperature.

As previously mentioned, the thermal provocation may in some embodiments conveniently be achieved by infrared radiation of the sites. In some embodiments, the infrared radiation of the sites may be achieved by a radiation source, typically part of the tissue imaging system, e.g. a single radiation source, with a respective collimator for each of the sites, optionally combined with filters.

Alternatively, in some embodiments, the thermal provocation may be achieved by a transparent heating device in contact with the skin of the site, e.g. in the form of a transparent plastic patch with embedded electrical heating threads, for enabling imaging of the site through the heating device. For instance, the heating device may be multi-spectrally transparent, i.e. transparent to the wavelengths of the tissue imaging system so that the blood circulatory response can be imaged through the heating device, and so that it can even be monitored during the thermal provocation.

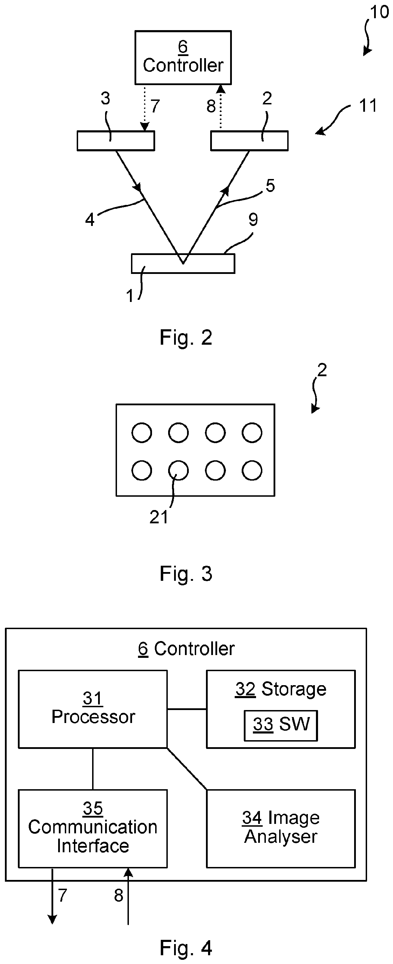

In some embodiments, the first and second imaging comprises Spatial Frequency Domain Imaging (SFDI) and/or Laser Speckle Contrast Imaging (LSCI). In some cases SFDI may be preferred and some examples of SFDI are discussed in relation to figures 2 and 3 (below).

In some embodiments, heat provocation may be followed by arterial occlusion, e.g. using a cuff, for instance a cuff around the upper arm of the subject if the target area is on the lower arm or palm of the hand of the same arm. Typically, the thermal provocation is performed before the occlusion, since the response to the thermal provocation is local but the occlusion affects a larger part of the subjects body (e.g. entire arm). If occlusion is instead performed first, a relatively long recovery time may be needed before the thermal provocation.

In some embodiments, the method is performed for both a first target area of the skin and a second target area of the skin, e.g. of the inside of the forearm and of the palm of the hand attached thereto. The method may then further comprise comparing the determined skin blood circulation response of the first target area (e.g. forearm) with the determined skin blood circulation response of the second target area (e.g. hand palm). A more complete study of the skin blood circulation may thus be achieved.

It is to be noted that any feature of any of the aspects may be applied to any other aspect, wherever appropriate. Likewise, any advantage of any of the aspects may apply to any of the other aspects. Other objectives, features and advantages of the enclosed embodiments will be apparent from the following detailed disclosure, from the attached dependent claims as well as from the drawings.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. The use of "first", "second" etc. for different features/components of the present disclosure are only intended to distinguish the features/components from other similar features/components and not to impart any order or hierarchy to the features/components.

### BRIEF DESCRIPTION OF THE DRAWINGS

Embodiments will be described, by way of example, with reference to the accompanying drawings, in which:
Fig 1 is a schematic side view of a tissue imaging system, in accordance with some embodiments of the present invention.
Fig 2 is a schematic side view of some parts of an example tissue imaging system, in accordance with some embodiments of the present invention.
Fig 3 is a schematic bottom view of a detector of a tissue imaging device, in accordance with some embodiments of the present invention.
Fig 4 is a schematic block diagram of a controller of a tissue imaging system, in accordance with some embodiments of the present invention.
Fig 5 is a schematic flow chart of some embodiments of the method of the present invention.

### DETAILED DESCRIPTION

Embodiments will now be described more fully hereinafter with reference to the accompanying drawings, in which certain embodiments are shown. However, other embodiments in many different forms are possible within the scope of the present disclosure. Rather, the following embodiments are provided by way of example so that this disclosure will be thorough and complete, and will fully convey the scope of the disclosure to those skilled in the art. Like numbers refer to like elements throughout the description.

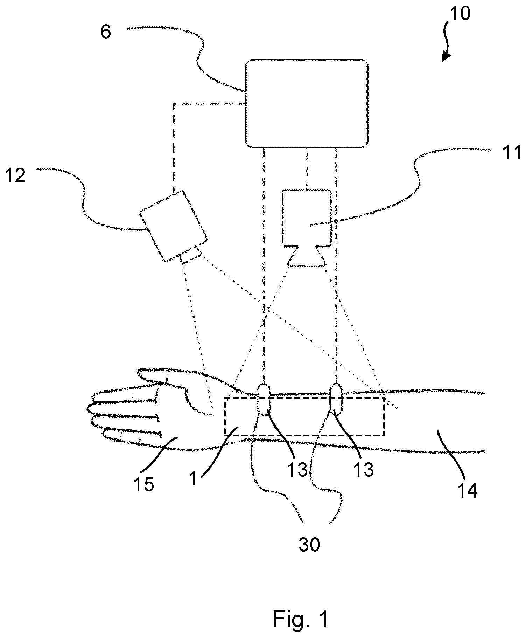

Figure 1 illustrates a tissue imaging system 10 for studying hemodynamics in live skin tissue of a target area 1 of a subject, here on the inside of a forearm 14 of the subject. Additionally or alternatively, in some other embodiments, the target area 1 may be on the palm of the hand 15 of the subject. At least two sites 13 within the target area are subjected to thermal provocation by heating to respective predetermined temperatures, e.g. by one or respective heating device(s) 30. Alternatively to heating devices 30 in contact with the skin at the sites 13, contactless heating device(s) 30, e.g. by infrared light, may be used.

The tissue imaging system 10 comprises a tissue imaging device 11 for performing the imaging of the target area 1, e.g. in accordance with SFDI and/or LSCI. The imaging by the tissue imaging device 11 may e.g. be continuous, whereby the first and second imaging follow each other directly as part of a same imaging stream, or intermittent.

In some embodiments, the tissue imaging system 10 also comprises a thermal camera 12 for detecting the skin temperature of the target area 1 and/or the sites 13 therein, which may e.g. be used for regulating the skin temperature at the sites 13, selecting the target area 1 and/or the at least two sites 13 prior to performing the first imaging, and/or for normalizing a blood property derivable from the first and second imaging data with regard to temperature, e.g. wherein the property is at least one of perfusion, hemoglobin concentration, oxygenation.

The tissue imaging system 10 also comprises a controller 6, which may e.g. control the tissue imaging device 11, the thermal camera 12 and/or the heating device(s) 30, and receive data such as imaging data from the tissue imaging device 11 and/or thermal data from the thermal camera 12. The controller 6 may also be arranged to determine the skin blood circulation response to the thermal provocation in the target area 1.

The tissue imaging device 11 may include various types of imaging systems, such as spatial frequency domain imaging (SFDI), diffused reflectance spectroscopy (DRS), Raman spectroscopy, and many others. Any suitable illumination may also be included in the tissue imaging device 11, e.g. a light engine or projector. The tissue imaging device 11 is configured to perform a first imaging and a second imaging of the target area 1 of the subject. This can provide information on both pre- and post-thermal-provocation, allowing for an assessment of changes in the target area 1 due to the provocation.

The thermal camera 12 may be an infrared camera for thermal imaging that can capture thermal imaging data of the target area 1. Specifications for the thermal camera may include a temperature range of 0 to 60°C, accuracy of ±2°C or better, and/or a frame rate of at least 30 frames per second. The thermal camera 12 may be used to monitor the temperature of the target area 1, and especially the sites 13 therein, throughout the diagnostic process, providing information on the thermal-provocation and allowing for accurate temperature control during the provocation.

The controller 6 may be configured to control the tissue imaging system 10. The controller 6 may ensure that the tissue imaging device 11 and thermal camera 12 are synchronized during the diagnostic process and may receive imaging data from the tissue imaging device 11 and the thermal camera 12. The controller 6 may also control the heating device(s) 30 based on the imaging data, allowing for precise temperature control during the method.

The heating devices 30 can include various types of devices, such as resistor-based probes, thermoelectric probes (like Peltier-based probes), contactless heat provocation devices (such as for infrared radiation), and others. The heating devices 30 may be configured to perform thermal-provocation at the two or more sites 13 of the target area 1, e.g. with temperature accuracy of ±0.5°C or less, a temperature control range of 0°C to 60°C, and/or a site 13 size of at least 10m by 10 mm, for example.

Figure 2 illustrates an SFDI tissue imaging device 11, in an example of a tissue imaging system 10, comprising an irradiator 3 and a detector 2. The irradiator may typically be a light source, e.g. comprising a plurality of Light-Emitting Diodes (LED), for instance emitting light 4 in the visible spectrum (VIS) with wavelengths from 380 nm to 750 nm and/or in the near-infrared spectrum (NIR) with wavelengths from 750 nm to 1400 nm. Additionally, the irradiator may comprise an infrared light source for contactless heating of the sites 13, as part of the heating device(s) 30. However, other wavelengths of electromagnetic radiation 4 may additionally or alternatively be emitted by the irradiator 3. For instance, at least five or at least eight LEDs may be comprised in the irradiator 3 for emitting the light 4, each with a respective different range of emitted wavelengths. The irradiator 3 preferably emits electromagnetic radiation, preferably VIS and NIR light, covering all the respective wavelength ranges of the cameras 21 in the detector 2 (see figure 3). To study the inside of the target area 1, and not only the surface 9 thereof, it may be desirable to only detect, by the detector 2, radiation 5 which has been reflected by diffuse reflection, some distance below the surface 9 of the target area, and not detect any radiation reflected by specular reflection at the surface 9. In some embodiments, the irradiator 3 is configured to irradiate the target area 1 with polarized light, e.g. linearly polarized. Since specular reflected radiation keeps its polarization, it may be removed, by a polarization filter or the like, before reaching the sensors, e.g. CCD sensors, of the cameras 21 of the detector 2.

The controller 6 may control the system 10 and for analysing the imaging data acquired by the cameras 21 of the detector 2 of the tissue imaging device 11. Thus, the controller 6 may control the irradiating with predetermined and calibration patterns by the irradiator 3 by sending control signals 7 to the irradiator 3, and the controller may receive image signals 8, comprising the digital images acquired by the detector 2 during the irradiating. The controller 6 is further discussed with reference to figure 4.

Figure 3 illustrates a detector 2, e.g. as discussed with reference to figure 2. The detector comprises a plurality of cameras 21, e.g. an array of cameras 21. Since the cameras 21 are arranged beside each other, each has a slightly different view of the sample 1, why the aligning of the present invention is desirable in order to determine where in respective images of the different cameras a point of the sample 1, e.g. a point on the surface 9, is found. Any number of cameras 21 may be comprised in the detector 2, e.g. within the range of 4-10 cameras 21, such as eight cameras as in the figure.

Each camera 21 is configured to detect radiation within a predefined wavelength range specific for that camera, typically distinct or not overlapping with respective wavelength ranges of the other cameras in the detector 2. The radiation detected by each camera is, or at least includes, electromagnetic radiation from diffuse reflection in the target area 1 as a result of the irradiating by the irradiator 3. The specific wavelength ranges may include at least one VIS wavelength range and/or at least one NIR wavelength range, preferably at least one VIS wavelength range and at least one NIR wavelength range. In some embodiments, the specific wavelength ranges include a green VIS wavelength range, a red VIS wavelength range, and at least one NIR wavelength range. To achieve these respective specific wavelength ranges of the cameras 21, each camera may be provided with a bandpass filter corresponding to its specific wavelength range. In some embodiments, the respective bandpass filters of the cameras 21 include bandpass filter(s) having a nominal centre-wavelength value of: 532 nm, 560 nm, 580 nm, 589 nm, 650 nm, 725 nm, 850 nm and/or 975 nm.

As mentioned above, the irradiating by the irradiator 3 may be by polarized light, whereby radiation reflected by specular reflection in the surface 9 of the target area 1 may be removed by a polarization filter, e.g. a polarization filter per camera 21. Thus, in some embodiments, each camera 21 is provided with a respective polarization filter.

In an example, all LEDs of the irradiator 3 radiate simultaneously when irradiating the target area 1. With the help of mirrors that reflect or transmit depending on wavelength, a common optical beam path may be formed. The beam may be polarized, typically by linear polarization, and shines on a DMD (digital micromirror device - a projector). Each camera 21 has a bandpass filter to acquire images in a specific wavelength range, as well as a polarizing filter to remove specular reflection.

In some embodiments, the thermal or infrared (IR) camera 12 may be included in the detector 2, e.g. as one of the plurality of camera 21 simultaneously acquiring an image of the target area 1. IR may be defined as wavelengths of from 1,4 micrometres (µm) to 15 µm.

Figure 4 illustrates an embodiment of the controller 6. The controller 6 comprises processing circuitry 31 e.g. a central processing unit (CPU). The processing circuitry 31 may comprise one or a plurality of processing units in the form of microprocessor(s). However, other suitable devices with computing capabilities could be comprised in the processing circuitry 31, e.g. an application specific integrated circuit (ASIC), a field programmable gate array (FPGA) or a complex programmable logic device (CPLD). The processing circuitry 31 is configured to run one or several computer program(s) or software (SW) 33 stored in a storage 32 of one or several storage unit(s) e.g. a memory. The storage unit is regarded as a computer readable means 32, forming a computer program product together with the SW 33 stored thereon as computer-executable components and may e.g. be in the form of a Random Access Memory (RAM), a Flash memory or other solid state memory, or a hard disk, or be a combination thereof. The processing circuitry 31 may also be configured to store data in the storage 32, as needed. The controller 6 may also comprise an image analyser 34, e.g. for determining the skin blood circulation response to the thermal provocation in the target area 1 based on the first and second imaging data. The controller 6 may also comprise a communication interface 35, e.g. for communicating with other parts of the imaging system 10. For example, the controller may send control signals 7 via the communication interface 35 and/or receive image signals 8 via the communication interface 35.

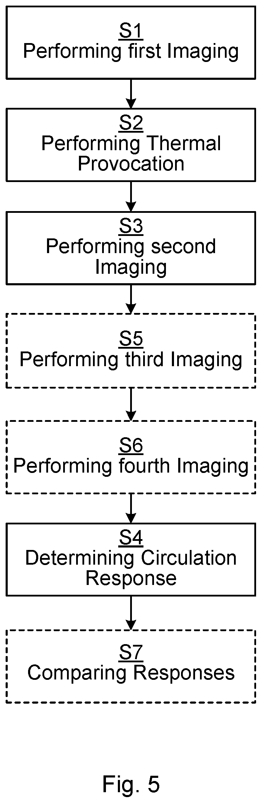

Figure 5 illustrates some embodiments of the method of the present invention. The method is for studying hemodynamics in live skin tissue of a subject. The method comprises performing a first imaging S1 of a target area 1 of the skin using the tissue imaging system 10 to generate first imaging data. The method also comprises performing, in parallel, at each of at least two sites 13 within said imaged S1 target area 1, thermal provocation S2 including heating the skin of the site 13 to its predetermined temperature. The method also comprises, during or after the thermal provocation S2, performing a second imaging S₃ of the target area 1 using said tissue imaging system 10 to generate second imaging data. The method also comprises determining S₄ the skin blood circulation response to the thermal provocation S2 in the target area 1 based on the first and second imaging data.

In some embodiments of the present invention, the method also comprises, after the performing of the second imaging S₃, during arterial occlusion at the target area, performing a third imaging S₅ of the target area 1 using said tissue imaging system 10 to generate third imaging data; and when releasing the arterial occlusion, performing a fourth imaging S6 of the target area 1 using said tissue imaging system 10 to generate fourth imaging data. Then, the determining S₄ of the skin blood circulation response may also based on said third and fourth imaging data.

Additionally or alternatively, in some embodiments of the present invention, the method (method steps S1, S2, S₃ and S₄, and optionally also steps S₅ and S6) may be performed for each of a first target area 1 of the skin (e.g. of the inside of the subjects forearm 14) and a second target area 1 of the skin (e.g. of the palm of the subjects hand 15 attached to said forearm 14). Then, the method may further comprise comparing S₇ the determined S₄ skin blood circulation response of the first target area 1 with the determined S₄ skin blood circulation response of the second target area 1.

### Examples

### 1. Tissue Imaging Device 11

### 1.1. Types of Imaging Techniques

The tissue imaging device 11 may comprise various types of imaging techniques, each with its unique advantages and capabilities. These imaging techniques can be used individually or in combination to provide a comprehensive and detailed diagnosis of the target area.

### 1.1.1. Spatial Frequency Domain Imaging (SFDI)

In some examples, the tissue imaging device 11 may comprise a spatial frequency domain imaging (SFDI) system. SFDI is a non-invasive imaging technique that uses structured illumination patterns to probe the optical properties of biological tissues. This technique can provide quantitative information on tissue absorption and scattering properties, which can be used to assess blood properties such as hemoglobin concentration, oxygen saturation levels, and blood flow rate. The use of SFDI in the tissue property imaging system 10 can provide high-resolution images of the target area, enabling a detailed assessment of the micro-circulation response to thermal-provocation.

### 1.1.2. Diffused Reflectance Spectroscopy (DRS)

In some examples, the tissue imaging device 11 may comprise a diffused reflectance spectroscopy (DRS) system. DRS is a non-invasive optical technique that measures the absorption and scattering properties of biological tissues by analyzing the diffusely reflected light. This technique can provide information on tissue composition, such as water content, lipid content, and blood properties like hemoglobin concentration and oxygen saturation levels. The use of DRS in the tissue property imaging system 10 can provide valuable information on the target area's tissue composition, aiding in the assessment of the micro-circulation response to thermal provocation.

### 1.1.3. Raman Spectroscopy

In some examples, the tissue imaging device 11 may comprise a Raman spectroscopy system. Raman spectroscopy is a non-invasive optical technique that measures the inelastic scattering of light by molecules in biological tissues. This technique can provide information on the molecular composition of tissues, such as the presence of specific biomolecules and their concentrations. The use of Raman spectroscopy in the tissue property imaging system 10 can provide detailed information on the target area's molecular composition, which can be useful in assessing the micro-circulation response to thermal-provocation and diagnosing vascular pathology.

### 1.1.4. Photoacoustic Tomography (PAT)

In some examples, the tissue imaging device 11 may comprise a photoacoustic tomography (PAT) system. PAT is a non-invasive imaging technique that combines the advantages of optical and ultrasound imaging to provide high-resolution images of biological tissues. This technique can provide information on tissue optical properties, such as absorption and scattering, as well as structural information like vessel diameter and blood flow rate. The use of PAT in the tissue property imaging system 10 can provide high-resolution images of the target area, enabling a detailed assessment of the micro-circulation response to thermal-provocation and diagnosing vascular pathology. Additionally or alternatively, multi- or hyperspectral imaging may be used.

### 1.2. Imaging Frequency and Field of View

In some examples, the tissue imaging device 11 may be configured to have an imaging frequency of greater than 0.5 Hz. This high imaging frequency can provide real-time information on the changes in the target area due to thermal-provocation, enabling a more accurate assessment of the micro-circulation response. Additionally, the tissue imaging device 11 may have a field of view that is at least partially overlapping with the field of view of the thermal camera 12. This overlapping field of view can ensure that the tissue imaging device 11 and the thermal camera 12 are synchronized during the diagnostic process, providing accurate temperature control during the thermal-provocation and a comprehensive assessment of the target area.

### 2. Thermal Camera and Imaging

In one example, the thermal camera 12 and imaging component of the tissue imaging system 10 comprises an infrared camera for thermal imaging. The infrared camera is configured to capture thermal imaging data of the target area 1 during the diagnostic process. This thermal imaging data provides valuable information on the temperature variations within the target area, allowing for accurate temperature control during the thermal provocation and enabling the identification of areas with increased blood flow or circulation changes.

### 2.1. Temperature Range and Accuracy

In one example, the temperature range of the thermal camera 12 is configured to cover a range of 0 to 60°C, which is suitable for capturing temperature variations within the human body. The accuracy of the thermal camera is ±2°C or better, ensuring that the temperature measurements are reliable and precise. This high level of accuracy is good for determining the micro-circulation response to the thermal-provocation and providing valuable information regarding hemodynamics and vascular pathology.

### 2.2. Frame Rate and Field of View

In some examples, the thermal camera 12 has a frame rate of at least 30 frames per second, allowing for real-time monitoring of the temperature variations within the target area 1. This high frame rate ensures that the thermal imaging data is captured continuously and accurately, providing a detailed and dynamic representation of the temperature changes during the diagnostic process.

The field of view of the thermal camera 12 may be configured to image a target area 1 of at least 10 cm by 10 cm at up to 1 meter range. This field of view allows for the capture of thermal imaging data from a large area of the subject body, providing a comprehensive and detailed assessment of the temperature variations within the target area 1.

### 2.3. Overlapping Fields of View with Tissue Imaging System 10

In one example, the field of view of the thermal camera 12 is at least partially overlapping with the field of view of the tissue imaging device 11. This overlapping field of view enables the simultaneous capture of thermal imaging data and tissue property imaging data, providing a more comprehensive and detailed assessment of the target area 1 during the method.

The overlapping fields of view may also facilitate the use of thermal imaging data to select sites 13 for thermal provocation, e.g. avoiding areas on top of large arteries, for example. Additionally, the thermal imaging data may be used to normalize baseline data of perfusion, hemoglobin, oxygenation, or combinations thereof, providing a more accurate and reliable assessment of the micro-circulation response to the thermal provocation.

### 3. Heating device(s) 30

Heating devices 30 are designed to perform thermal-provocation at two or more sites 13 of the target area 1 in parallel, allowing for a comprehensive assessment of the micro-circulation response to the provocation.

### 3.1. Types of Heating Devices

In some examples, the heating deviceds 30 may comprise resistor-based probes. These probes utilize resistive heating elements to generate heat, which is then transferred to the target area through direct contact. Resistor-based probes offer a simple and reliable method for thermal-provocation, with the advantage of easy temperature control and accurate temperature measurements.

In other examples, the heating devices 30 may comprise thermoelectric probes, such as Peltier-based probes. These probes utilize the Peltier effect to generate heat or cooling by applying a voltage across a thermoelectric material. Peltier-based probes offer precise temperature control and the ability to switch between heating and cooling modes, providing a versatile option for thermal-provocation.

In yet other examples, the heating devices 30 may comprise contactless heat provocation devices, such as for infrared radiation. These devices 30 generate heat through the emission of infrared radiation, which is absorbed by the sites 13 without the need for direct contact. Contactless heat provocation offer the advantage of avoiding potential discomfort or skin irritation associated with direct contact probes, while still providing effective thermal-provocation.

### 3.2. Temperature Control and Accuracy

The heating devices 30 are designed to provide accurate and precise temperature control during the method. In one example, the devices 30 have a temperature accuracy of ±0.5°C or less, ensuring that each predetermined temperature is achieved and maintained throughout the heat provocation. The temperature control range of the devices 30 may be between 0°C to 60°C, providing a wide range of temperatures for various applications.

### 3.3. Site 13 Size and Shape Adaptability

In some examples, the heating devices 30 may provide a heat provocation site 13 size of at least 10 mm by 10 mm, or even at least 15 mm by 15 mm, ensuring that a sufficient site area of the target area 1 is subjected to thermal provocation. The devices 30 may also be designed for adaptable site shapes, allowing them to conform to various anatomical features and ensuring effective heat transfer to the sites 13.

### 3.4. Multi-spectrally Transparent Heating Devices 30

In one example, the heating devices 30 may be multi-spectrally transparent, allowing the full sequence of events to be imaged in the provoked site 13. This feature provides the advantage of enabling continuous imaging of the target area 1 during the thermal provocation process, allowing for a more accurate assessment of the micro-circulation response and a more comprehensive diagnostic evaluation. An example of continuous imaging of the target area may comprise a continuous recorded video of the response to the heating.

### 4. Control System and Synchronization

Controller 6 may be configured to control the tissue imaging system 10. The controller 6 may ensure that the imaging device 11 and thermal camera 12 are synchronized during the method, providing accurate and consistent imaging data for analysis. This synchronization allows for reliable and meaningful results, as it allows for the precise comparison of imaging data before and after thermal-provocation.

### 4.1. Controlling Tissue Imaging Device and Heating Devices

In some examples, the controller 6 is responsible for controlling the tissue imaging device 11 and the heating devices 30. This control may include adjusting the imaging frequency, field of view, and other imaging parameters of the tissue imaging device 11, as well as controlling the temperature, size, shape, and other characteristics of the heating devices 30. By controlling these components, the controller 6 ensures that the method is performed accurately and efficiently, providing high-quality imaging data for analysis.

### 4.2. Receiving Imaging Data and Adjusting Heating Devices

Controller 6 receives imaging data from the tissue imaging device 11 and the thermal camera 12. Based on this imaging data, the controller 6 may adjust the heating devices 30 accordingly. For instance, the controller 6 may use the thermal imaging data to regulate the temperature at the sites 13, ensuring that the predetermined temperatures are maintained. This precise temperature control may be essential for obtaining reliable and meaningful results, as it allows for the accurate assessment of the micro-circulation response to the thermal provocation.

### 4.3. Blood Occlusion Device Control

In some examples, the controller 6 may also control a blood occlusion device applied to a part of the subject relevant for the target area 1. The blood occlusion device may comprise a blood pressure cuff, a tourniquet, or an automated cuff, for example. The controller 6 may control the blood occlusion device based on the imaging data obtained from the tissue imaging device 11 and/or the thermal camera 12. For instance, the controller 6 may adjust the cuff-occlusion pressure in dependence on imaging data of the target area 1, such as haemoglobin concentration, water concentration, temperature profile, or perfusion. By controlling the blood occlusion device, the controller 6 can further study the hemodynamics, providing additional information and a more comprehensive assessment of the subject.

An advantages of the controller 6 and its synchronization capabilities include the ability to obtain accurate and consistent imaging data, precise temperature control during thermal provocation, and the potential for additional diagnostic information through blood occlusion device control. These advantages contribute to a more reliable and comprehensive assessment of hemodynamics.

### 5. Method of Use and Imaging Sequence

In one example, the method of use and imaging sequence for hemodynamics involves several steps, including baseline measurement and blood properties, thermal-provocation and imaging, blood occlusion and imaging, and micro-circulation response determination.

### 5.1. Baseline Measurement and Blood Properties

The method begins with performing a first imaging S1 of the target area 1 of the subject using the tissue property device 11. This first imaging provides baseline measurements of the target area, such as blood properties (e.g., hemoglobin concentration, oxygen saturation levels, blood flow rate, vessel diameter), and hemodynamics (changes in blood properties over time). The baseline measurement allows for an assessment of the initial state of the target area 1 before any thermal provocation is applied, which is essential for determining the micro-circulation response to the provocation.

### 5.2. Thermal-Provocation and Imaging

The method proceeds with performing thermal provocation S2 at two or more sites 13 of the target area 1 using heating devices 30. The heating devices 30 may be multi-spectrally transparent, allowing for the full sequence of events to be imaged in the sites 13. The use of different temperatures at different sites 13 within the target area 1 (e.g., between 30 degrees and 40 degrees) may provide a more comprehensive assessment of the micro-circulation response to the provocation S2.

After the thermal-provocation S2, a second imaging S3 is performed of the target area 1 using the tissue imaging device 11. In some examples, the second imaging is continuous to the first imaging, allowing for a seamless assessment of the changes in the target area 1 due to the thermal provocation S2. The thermal camera 12 may be used to capture thermal imaging data of the target area 1 during the second imaging S3, providing information on the thermal provocation and allowing for accurate temperature control during the provocation.

### 5.3. Blood Occlusion and Imaging

In some examples, the method may further comprise applying a blood occlusion device to a part of the subject relevant for the target area 1. The blood occlusion device may be controlled by the controller 6 and may include any type, such as a blood pressure cuff, tourniquet, or automated cuff. The application of the blood occlusion device allows for the study of hemodynamics under different conditions, such as arterial occlusion.

While maintaining blood occlusion, a third imaging S₅ of the target area 1 may be performed using the tissue imaging device 11. The third imaging may be performed for a duration of 3-5 minutes, providing information on the micro-circulation response within the target area 1 during the occlusion. In some examples, the pressure from the blood occlusion device may be released, and a fourth imaging S6 of the target area 1 may be performed using the tissue imaging device 11. One or more images may be obtained over a period of up to 15 minutes, providing further information on the micro-circulation response within the target area 1 after the occlusion.

### 5.4. Micro-circulation Response Determination

In some examples, the method concludes with determining S₄ a micro-circulation response to the thermal provocation within the target area 1 based on the first imaging data, second imaging data, and optionally, the third and fourth imaging data. The micro-circulation response may be determined using various data analysis techniques, such as machine learning algorithms, statistical analysis techniques, image segmentation, feature extraction, clustering algorithms, time-series analysis, regression analysis, and frequency analysis. The analysis results can be used to determine the micro-circulation response to the thermal provocation and provide valuable information regarding hemodynamics and vascular pathology.

The method of use and imaging sequence may be performed at multiple target areas of a patient, such as the forearm, palm of the hand, soles of the feet, dorsal side of the feet, face, and legs. In some examples, the tissue property imaging system 10 may be used in combination with several areas of provocations with different temperatures or different vessel compositions, such as the palm of the hand versus the forearm, within the same field of view of the tissue property imaging system. This approach may provide a more comprehensive and detailed diagnosis, as well as potentially speeding up the examination process.

### 6. Data Analysis and Diagnostic Assessment

In one example, the data analysis and diagnostic assessment process involve the use of various software algorithms and techniques to analyze the imaging data obtained from the tissue imaging device 11 and the thermal camera 12. This analysis may provide valuable insights into the micro-circulation response to the thermal provocation within the target area 1, ultimately aiding in the diagnosis of hemodynamics and vascular pathology.

### 6.1. Software Algorithms for Imaging Data Analysis

In some examples, the data analysis process may comprise the use of machine learning algorithms to identify patterns in the imaging data. These algorithms can be trained on a large dataset of imaging data from various subjects and conditions, allowing them to recognize and classify patterns that may be indicative of specific hemodynamic responses or vascular pathologies.

In other examples, statistical analysis techniques may be applied to compare and evaluate differences in the imaging data before and after thermal-provocation. These techniques can help to identify significant changes in blood properties, oxygen saturation levels, blood flow rate, vessel diameter, and other relevant parameters, providing a quantitative assessment of the micro-circulation response.

### 6.2. Temperature Monitoring and Blood Flow Changes

In one example, the thermal camera 12 may be used to monitor temperature variations within the target area 1 during thermal provocation. This can provide real-time feedback on the effectiveness of the provocation and help to ensure accurate temperature control.

In some examples, the imaging data obtained from the tissue imaging device 11 and the thermal camera 12 may be used to identify areas of increased blood flow or circulation changes within the target area 1. This can provide valuable information on the micro-circulation response to the thermal provocation, as well as help to identify any potential abnormalities or pathologies within the vascular system.

### 6.3. Comprehensive Diagnostic Assessment with Multiple Target Areas

In one example, the method may be performed at multiple target areas 1 of the subject, such as the forearm, palm of the hand, soles of the feet, dorsal side of the feet, face, and/or legs. This can provide a more comprehensive assessment of the subject's hemodynamics, as well as help to identify any potential differences or abnormalities between different areas of the body.

In other examples, the tissue imaging system 10 may be used in combination with several areas 1 or body sites 13 with different vessel compositions, such as the palm of the hand versus the forearm, within the same field of view of the tissue imaging device 11. This can enable the study of different vessel compositions and their responses to thermal provocation, providing valuable insights into the underlying hemodynamics.

The present disclosure has mainly been described above with reference to a few embodiments. However, as is readily appreciated by a person skilled in the art, other embodiments than the ones disclosed above are equally possible within the scope of the present disclosure, as defined by the appended claims.

## Claims

1. A method for studying hemodynamics in live skin tissue of a subject, the method comprising:
performing a first imaging (S1) of a target area (1) of the skin using a tissue imaging system (10) to generate first imaging data;
in parallel, at each of at least two sites (13) within said imaged (S1) target area (1), performing thermal provocation (S2) including heating the skin of the site (13) to a predetermined temperature;
during or after the thermal provocation (S2), performing a second imaging (S₃) of the target area using said tissue imaging system (10) to generate second imaging data; and
determining (S₄) a skin blood circulation response to the thermal provocation (S2) in the target area (1) based on the first and second imaging data.

2. The method of claim 1, wherein the respective predetermined temperatures are different to each other.

3. The method of any preceding claim, wherein the thermal provocation (S2) also includes regulating the skin temperature of the site (13) to remain at said predetermined temperature for the duration of the thermal provocation (S2).

4. The method of claim 3, wherein the regulating is based on real-time thermal data from a thermal camera (12) detecting the skin temperature of the site (13).

5. The method of any preceding claim, wherein thermal data from a thermal camera (12) is used for selecting the target area (1) and/or the at least two sites (13), prior to performing the first imaging (S1).

6. The method of any preceding claim, wherein thermal data from a thermal camera (12) is used for normalizing a blood property derivable from the first and second imaging data, with regard to temperature, e.g. wherein the property is at least one of perfusion, hemoglobin concentration, oxygenation.

7. The method of any preceding claim, wherein the thermal provocation (S2) is achieved by infrared radiation of the sites (13).

8. The method of claim 7, wherein the infrared radiation of the sites (13) is achieved by a single radiation source (3) with a respective collimator for each of the sites (13), optionally combined with filters.

9. The method of any claim 1-6, wherein the thermal provocation (S2) is achieved by a transparent heating device (30) in contact with the skin of the site (13).

10. The method of any preceding claim, wherein the first and second imaging (S1, S3) comprises Spatial Frequency Domain Imaging, SFDI, and/or Laser Speckle Contrast Imaging, LSCI, preferably SFDI.

11. The method of any preceding claim, further comprising, after the performing of the second imaging (S₃):
during arterial occlusion at the target area, performing a third imaging (S₅) of the target area (1) using said tissue imaging system (10) to generate third imaging data; and
when releasing the arterial occlusion, performing a fourth imaging (S6) of the target area (1) using said tissue imaging system (10) to generate fourth imaging data;
wherein the determining (S₄) of the skin blood circulation response is also based on said third and fourth imaging data.

12. The method of any preceding claim, performed for both a first target area (1) of the skin and a second target area (1) of the skin, the method further comprising comparing (S₇) the determined (S₄) skin blood circulation response of the first target area with the determined (S₄) skin blood circulation response of the second target area.

13. A tissue imaging system (10) for performing the method of any preceding claim, the system comprising:
a tissue imaging device (11) for performing the imaging of the target area (1);
a thermal provocation device (30) for, in parallel, at each of the at least two sites (13) within the target area (1), performing the thermal provocation; and
a controller (6) comprising processing circuitry (31) and storage (32) storing instructions (33) executable by said processing circuitry whereby said controller is operative to determine the skin blood circulation response to the thermal provocation in the target area (1).

14. The system of claim 13, further comprising a thermal camera (12) for detecting skin temperature of the target area (1).

15. The system of claim 13 or 14, wherein the thermal provocation device (30) comprises an infrared radiation source (3) with a respective collimator for each site (13), optionally combined with filters.
